# EUROPÄISCHE PATENTSCHRIFT

(11) **EP 2 438 867 B1**
(45) Veröffentlichungstag und Bekanntmachung des Hinweises auf die Patenterteilung: **11.01.2017**
(21) Anmeldenummer: 11008013.2
(22) Anmeldetag: 04.10.2011
(51) Int. Cl.: A61B 17/32, A61B 17/3205

(54) **Medizinisches Instrument**
Medical instrument
Instrument médical

(30) Priorität: 05.10.2010 DE 102010037974
(43) Veröffentlichungstag der Anmeldung: 11.04.2012
(73) Patentinhaber: Trokamed GmbH, 78187 Geisingen (DE)
(72) Erfinder: Tröndle, Karlheinz, 78187 Geisingen (DE)
(74) Vertreter: Patentanwälte und Rechtsanwalt Weiß, Arat & Partner mbB

(56) Entgegenhaltungen:
- EP-A2- 0 858 774
- EP-A2- 1 598 023
- WO-A1-2005/060842
- WO-A1-2008/145380
- DE-U1-202006 006 914
- US-A- 5 569 284
- US-A- 5 814 044
- US-A1- 2003 225 344
- US-A1- 2006 111 725
- US-A1- 2007 083 081
- US-A1- 2007 173 873

## Beschreibung

Die Erfindung betrifft ein medizinisches Instrument zum Schneiden von biologischem, insbesondere menschlichem Gewebe entsprechend dem Oberbegriff von Anspruch 1.

### Stand der Technik

Obwohl es sich im vorliegenden Fall insbesondere um ein spezielles medizinisches Instrument handelt, welches auch unter dem Fachbegriff Morcellator" bekannt ist, soll darauf die Erfindung nicht beschränkt sein. Die Erfindung ist überall dort anzuwenden, wo über ein sich drehendes Schneidmodul, welches insbesondere als Schneidrohr ausgestaltet ist, eine Tätigkeit im Körper eines Lebewesens durchgeführt wird. Heute werden derartige medizinische Instrument vor allem bei endoskopischen Eingriffen verwendet. Sie dienen dazu, grössere Gewebeanteile zu entfernen.

Speziell der Morcellator weist als Schneidmodul ein Schneidrohr auf, welches am distalen Ende eine Schneide besitzt. Dieses Schneidrohr wird beispielsweise durch ein Endoskop in den Körper eingeführt und dann in Drehbewegung versetzt. Hierdurch entfernt die Schneide Gewebeanteile, die dann durch das Schneidrohr selbst aus dem Körper entnommen werden. Hierzu kann das Gewebeteil abgesaugt oder aber auch durch ein weiteres medizinisches Instrument entfernt werden, welches dann beispielsweise durch ein Ventilmodul hindurch in das Schneidrohr und durch dieses hindurch geführt wird. Ein derartiges medizinische Instrument ist beispielsweise in der DE 103 58 279 A1 und der WO 2005/060842 A1 beschrieben.

Aus der US 2003/0225344 A1 ist eine Vorrichtung zur Knochenmarksentnahme bekannt. Hierbei ist ein Schneidrohr in einem Gehäuse vorgesehen, wobei das Schneidrohr von einem Trokar durchsetzt ist. Das Schneidrohr selbst ist im Gehäuse drehbar angeordnet. Auf ihm sitzt ein Kegelrad, welches von einem Kegelrad angetrieben wird, das auf der Abtriebswelle eines Motors aufsitzt.

Aus der US 5 814 044 ist ein Morcellator bekannt, bei dem das Schneidrohr entfernbar in einem Handmodul eingesetzt ist. Dabei geht das Schneidrohr mit dem Handmodul eine Rastverbindung ein, wobei eine entsprechende Raste mit einem Rastnocken zusammenwirkt. Die Übertragung der Drehbewegung einer Abtriebswelle eines Rotors auf das Schneidrohr erfolgt über zwei Zahnräder, die miteinander kämmen.

### Aufgabe

Aufgabe der vorliegenden Erfindung ist es, das oben beschriebene medizinische Instrument handhabbarer und flexibel auszugestalten.

### Lösung der Aufgabe

Zur Lösung der Aufgabe die Merkmale des kennzeichnenden Teils von Anspruch 1.

Dies bedeutet, dass beispielsweise das Handmodul immer gleich bleiben kann, während das Schneidmodul ausgetauscht wird. Das Schneidmodul kann gegen ein gleichartiges Schneidmodul bei Verschleiss oder Verschmutzung ausgetauscht werden, denkbar ist aber auch der Einsatz unterschiedlich ausgestalteter Schneidmodule für unterschiedliche medizinische Tätigkeiten.

In einem bevorzugten Ausführungsbeispiel der Erfindung ist das Schneidmodul als ein hohles Schneidrohr ausgebildet, welches um seine Achse drehbar mit dem Handmodul verbunden ist. In diesem Fall bildet das medizinische Instrument einen Morcellator.

Die lösbare Verbindung zwischen Schneidmodul und Handmodul ist als Rastverbindung ausgestaltet.

Das bedeutet, dass das Schneidrohr einfach in das Handmodul eingesetzt ist und einen Rast- oder Klickverschluss eingeht. Ebenso kann das Schneidmodul aus dem Handmodul entfernt werden, indem entweder am Schneidmodul und/oder am Handmodul gezogen wird.

Eine Rastverbindung wird dadurch bewirkt, dass an einem Element eine bevorzugt als Rastfeder ausgebildete Raste angeordnet ist, die mit einem entsprechenden Rastnocken in dem Handmodul zusammenwirkt. Dabei kann die Raste bzw. Rastfeder selbst aus dem Schneidrohr herausgeformt sein und ist bevorzugt selbstfedernd ausgebildet, so dass sie beim Einsetzen des Schneidmoduls in das Handmodul nach innen nachgibt und dann den Rastnocken hinterschnappt. Ebenso gibt sie bei Herausziehen des Schneidmoduls aus dem Handmodul nach innen nach und überfährt den Rastnocken. Der Rastnocken kann dabei ringförmig als Innenring in einem Aufnahmeelement in dem Handmodul vorgesehen sein, jedoch sind auch andere Möglichkeiten denkbar.

Des weiteren soll dem Schneidmodul ein Aussenzahnkranz zugeordnet sein, der mit dem Innenzahnkranz in dem Handmodul zusammenwirkt. Dieser Innenzahnkranz steht über ein Ringkegelrad mit einem Kegelantriebsrad in Verbindung, welches wiederum bevorzugt auf einer Drehachse eines Motors aufsitzt. Hierdurch wird die Drehbewegung der Drehachse auf das Schneidmodul übertragen.

Zur Abstützung beim Drehen sind dem Schneidmodul bevorzugt zwei beabstandete Gleitlagerringe aufgesetzt, die in entsprechenden Gleitlagern in dem Aufnahmeelement in dem Handmodul drehen. Es ist vorgesehen , dass das Schneidmodul mit den Gleitringen in den Gleitlagern mit mindestens 0,2 Nm jedoch maximal 5 Nm lagert. Die Gleitringe sind kraftschlüssig auf das Schneidmodul aufgepresst, ferner ist daran gedacht, das Schneidmodul insbesondere in dem proximalen Bereich, der in das Handmodul eingesetzt wird, mehrteilig auszubilden.

Auf der dem Schneidmodul gegenüberliegenden Seite des Handmoduls ist bevorzugt eine Ventileinheit eingesetzt. Diese kann eine bajonettartige Verbindung mit dem Handmodul eingehen. In der Ventileinheit befindet sich ein Ventil, welches das Einsetzen eines weiteren medizinischen Instruments zulässt, jedoch dieses Instrument so luftdicht wie möglich umschliesst.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
**Figur 1** eine perspektivische Ansicht eines erfindungsgemässen medizinischen Instruments zum Schneiden von menschlichem Gewebe;

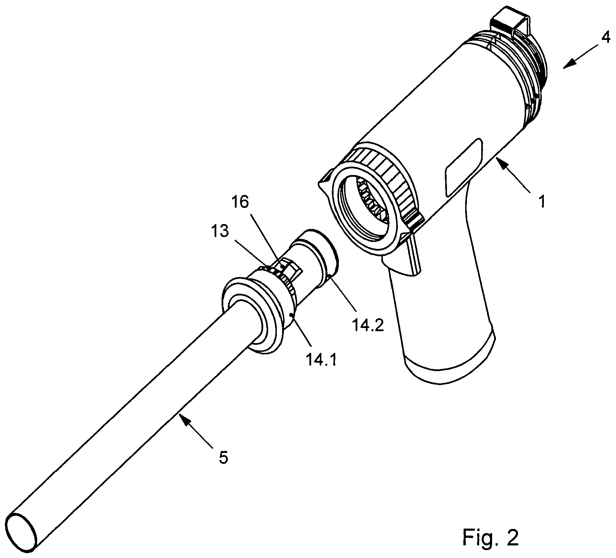

**Figur 2** eine perspektivische Ansicht des medizinischen Instruments gemäss Figur 1, teilweise in Explosionsdarstellung;

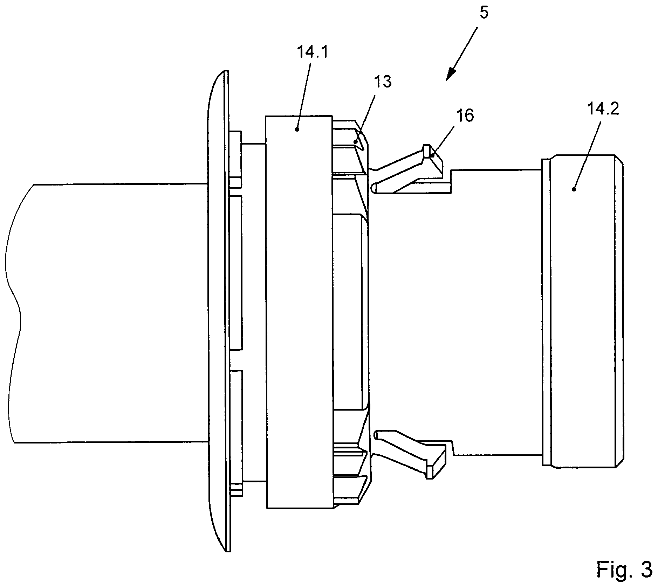

**Figur 3** eine Draufsicht auf einen Einschubbereich eines erfindungsgemässen Schneidrohres;
**Figur 4** einen teilweise dargestellten Längsschnitt durch ein Handmodul mit eingesetztem Schneidrohr;
**Figur 5** einen teilweise dargestellten Längsschnitt durch ein Ausführungsbeispiel eines distalen Endes eines Schneidrohres;
**Figur 6** einen teilweise dargestellten Längsschnitt durch ein weiteres Ausführungsbeispiel eines distalen Endbereichs eine Schneidrohres;
**Figur 7** einen teilweise dargestellten Längsschnitt durch ein weiteres Ausführungsbeispiel eines distalen Endbereichs eine Schneidrohres.

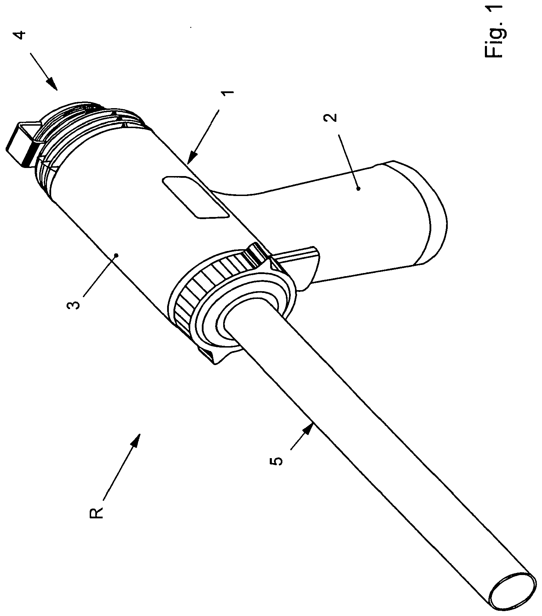

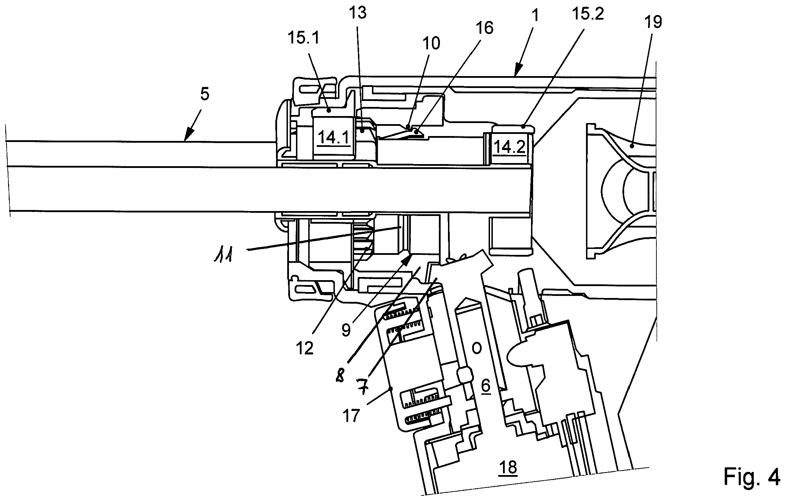

Gemäss Figur 1 weist ein erfindungsgemässes medizinisches Instrument R ein Handmodul 1 auf, welches aus einem Griffteil 2 und einem Führungsteil 3 besteht. In ein offenes Ende des Führungsteils 3 ist ein Ventilmodul 4 und in das andere offene Ende ein Schneidmodul 5 eingesetzt. In dem Griffteil 2 befindet sich ein in Figur 4 gezeigter Motor 18, auf dessen Drehachse 6 ein Kegelantriebsrad 7 aufgesetzt ist. Dieses Kegelantriebsrad 7 wirkt mit einem Ringkegelrad 8 zusammen, das ein Aufnahmeelement 9 in dem Führungsteil des Handmoduls 1 umfängt, wobei ein ringförmig ausgestalteter Rastnocken 10 in eine entsprechende Ringnut 11 in dem Aufnahmeelement 9 eingreift. Diese Ringnut 11 bildet in das Innere des Aufnahmeelements 9 wiederum die ringförmige Rastnocke 10 aus.

In Gebrauchslage gemäss Figur 4 übergreift das Ringkegelrad 8 mit einem Innenzahnkranz 12 einen Aussenzahnkranz 13, der auf das als Schneidrohr ausgebildete Schneidmodul 5 aufgesetzt ist. Hierdurch wird eine Drehbewegung des Kegelantriebsrads 7 über das Ringkegelrad 8 auf den Aussenzahnkranz 13 übertragen und das Schneidmodul 5 um seine Längsachse gedreht.

Zur Abstützung des Schneidmoduls 5 bei der Drehung um seine Längsachse sind dem Schneidmodul 5 beabstandet voneinander zwei Gleitringe 14.1 und 14.2 aufgesetzt, die wiederum in einem vorderen Gleitlager 15.1 und einem hinteren Gleitlager 15.2 drehen.

Des weiteren ist erkennbar, dass das Schneidmodul 5 über eine Rastverbindung lösbar in das Handmodul 1 bzw. das Aufnahmeelement 9 eingesetzt ist. Hierzu stehen von dem Schneidmodul 5 zwischen dem hinteren Gleitlager 15.2 und dem Aussenzahnkranz 13 Rastfedern 16 ab, die in Zusammenbaulage die nach innen als Rastnocke vorstehende Rastnut 11 hintergreifen.

Die Funktionsweise der vorliegenden Erfindung ist folgende:
Nach dem Einsetzen des Schneidmoduls 5 in beispielsweise einen menschlichen Körper zum Entnehmen von Gewebe, beispielsweise durch einen Trokar, wird über einen Druckknopf 17 der Motor 18 bzw. dessen Drehachse 6 in Drehbewegung versetzt. Dabei dreht auch das Kegelantriebsrad 7 und treibt über das Ringkegelrad 8, dessen Innenzahnkranz 12 und den Aussenzahnkranz 13 das Schneidmodul 5 an bzw. versetzt dieses in Drehbewegung. Dabei drehen die Gleitringe 14.1 und 14.2 in den entsprechenden Gleitlagern 15.1 und 15.2. Die Rastfedern 16 hintergreifen den in das Innere des Aufnahmeelements 9 vorspringenden ringförmigen Rastnocken. Sollte es notwendig sein, kann durch ein entsprechendes Ventil 19 in dem Ventilmodul 4 ein weiteres chirurgisches Instrument eingesetzt werden, welches dann auch durch das Schneidmodul 5 bis zu dessen distalem Ende hindurch geführt werden kann.

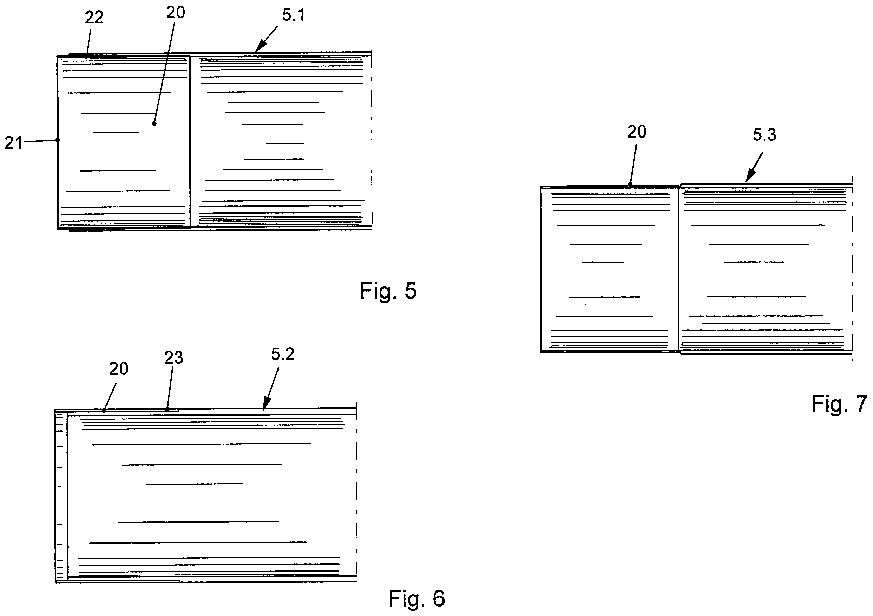

Erfindungsgemäss befindet sich am distalen Ende des Schneidmoduls 5 eine Schneide bzw. ein Schneidelement 20 mit einer Schneide 21. Dieses Schneidelement 20 ist im Rahmen der vorliegenden Erfindung aus Federstahl gebildet und zu einer Hülse zusammengerollt. Gemäss Figur 5 ist zur Aufnahme dieses Schneidelements 20 in einem entsprechenden Schneidmodul 5.1 ein innerer Einstich 22 ausgebildet, so dass das Schneidelement 20 in das Innere des Schneidmoduls 5.1 eingesetzt ist.

Gemäss Figur 6 weist ein Schneidmodul 5.2 einen äusseren Einschnitt 23 auf, in den das Schneidelement 20 eingesetzt ist. D.h., in diesem Fall umfängt das Schneidelement 20 das distale Ende des Schneidmoduls 5.2. Gemäss Figur 7 schlägt das Schneidelement 20 stumpf an ein Schneidmodul 5.3 an bzw. ist an dieses Schneidmodul 5.3 stumpf angeschweisst.

Ein erfindungsgemässes Ausführungsbeispiel des medizinischen Instruments ist derart gestaltet, dass das Schneidmodul 5 ein Schneidrohr ist, an welchem distal die Schneide 21 radial umfassend bzw. umlaufend angeordnet ist. Ausserdem ist die Schneide 21 stumpf an das Schneidmodul 5.3 angesetzt. Daneben ist die Schneide 21 in einen aussen oder innen dem distalen Ende des Schneidmoduls 5.2, 5.1 eingeformten Einstich 23, 22 eingesetzt. Zuletzt ist anderenends des Schneidmoduls 5 mit dem Handmodul 1 ein Ventilmodul 4 bajonettverschlussartig verbunden.

**Bezugszeichenliste**

| | | | | | |
|---|---|---|---|---|---|
| 1 | Handmodul | 34 | | 67 | |
| 2 | Griffteil | 35 | | 68 | |
| 3 | Führungsteil | 36 | | 69 | |
| 4 | Ventilmodul | 37 | | 70 | |
| 5 | Schneidmodul | 38 | | 71 | |
| 6 | Drehachse | 39 | | 72 | |
| 7 | Kegelrad | 40 | | 73 | |
| 8 | Rindkegelrad | 41 | | 74 | |
| 9 | Aufnahmeelement | 42 | | 75 | |
| 10 | Rastnocke | 43 | | 76 | |
| 11 | Ringnut | 44 | | 77 | |
| 12 | Innenzahnkranz | 45 | | 78 | |
| 13 | Aussenzahnkranz | 46 | | 79 | |
| 14 | Gleitring | 47 | | | |
| 15 | Gleitlager | 48 | | | |
| 16 | Rastfeder | 49 | | | |
| 17 | Druckknopf | 50 | | | |
| 18 | Motor | 51 | | | |
| 19 | Ventil | 52 | | | |
| 20 | Schneidelement | 53 | | | |
| 21 | Schneide | 54 | | | |
| 22 | innerer Einstich | 55 | | | |
| 23 | äusserer Einstich | 56 | | | |
| 24 | | 57 | | R | med. Instrument |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Medizinisches Instrument zum Schneiden von biologischem, insbesondere menschlichem Gewebe mittels einem Schneidmodul (5) mit einer Schneide (21), welches in ein Handmodul (1) eingesetzt ist, wobei das Schneidmodul (5) in dem Handmodul (1) lösbar festgelegt ist und eine Rastverbindung (10, 16) mit dem Handmodul (1) eingeht, wobei dem Schneidmodul (5) zumindest eine Raste (16) zugeordnet ist, die mit einem Rastnocken (10) in dem Handmodul (1) zusammenwirkt,
**dadurch gekennzeichnet,**
**dass** der Rastnocken (10) als Innenring in einem Aufnahmeelement (9) ausgebildet ist und auf dem Schneidmodul (5) ein Zahnkranz (13) angeordnet ist, der mit einem Innenzahnkranz (12) an dem Aufnahmeelement (9) zusammenwirkt

2. Medizinisches Instrument nach Anspruch 1, **dadurch gekennzeichnet, dass** auf dem Aufnahmeelement (9) ein Kegelrad (8) angeordnet ist, welches mit einem weiteren Kegelrad (7) zusammenwirkt, das auf einer Drehachse (6) eines Motors (18) aufgesetzt ist.

3. Medizinisches Instrument nach Anspruch 1 oder 2, **dadurch gekennzeichnet, dass** die zumindest eine Raste (16) aus dem Schneidrohr herausgeformt und selbstfedernd ausgebildet ist.

4. Medizinisches Instrument nach wenigstens einem der Ansprüche 1 bis 3, **dadurch gekennzeichnet, dass** das Schneidmodul (5) ein hohles Schneidrohr ist, welches um seine Drehachse drehbar mit dem Handmodul (1) verbunden ist.

5. Medizinisches Instrument nach wenigstens einem der Ansprüche 1 bis 4, **dadurch gekennzeichnet, dass** auf das Schneidmodul (5) zumindest ein, bevorzugt zwei beabstandete Gleitlager-Ringe (24.1, 14.2) aufgesetzt ist/sind, die mit einem bzw. zwei Gleitlagern (15.1, 15.2) in dem Handmodul (1) zusammenwirkt bzw. - wirken.

6. Medizinisches Instrument nach Anspruch 5, **dadurch gekennzeichnet, dass** in dem Handmodul (1) zumindest ein Gleitlager (15.1, 15.2) vorgesehen ist, das auf Gleitringen (14.1, 14.2) auf dem Schneidmodul (5) mit einem definierten Reibmoment von mindestens 0,2 Nm jedoch maximal von 5 Nm angeordnet ist.

7. Medizinisches Instrument nach Anspruch 6, **dadurch gekennzeichnet, dass** die Gleitlager-Ringe (14.1, 14.2) kraftschlüssig auf das Schneidmodul (5) aufgepresst sind.

## Claims

1. A medical instrument for cutting biological, in particular human, tissue by means of a cutting module (5) with a cutting edge (21), which cutting module is inserted into a hand module (1), wherein the cutting module (5) is detachably secured in the hand module (1) and enters into a locking connection (10, 16) with the hand module (1), wherein at least one catch (16) is associated with the cutting module (5) and cooperates with a catching protrusion (10) in the hand module (1), **characterised in that** the catching protrusion (10) is in the form of an inner ring in a receiving element (9) and a toothed wheel (13) is arranged on the cutting module (5), which toothed wheel (13) cooperates with an inner toothed wheel (12) at the receiving element (9).

2. A medical instrument according to claim 1, **characterised in that** a bevel gear (8) is arranged on the receiving element (9), which bevel gear (8) cooperates with another bevel gear (7) placed on a rotational shaft (6) of a motor (18).

3. A medical instrument according to claim 1 or 2, **characterised in that** the at least one catch (16) is formed from the cutting tube and is inherently resilient.

4. A medical instrument according to at least one of claims 1 to 3, **characterised in that** the cutting module (5) is a hollow cutting tube connected in a manner rotatable about its rotational axis to the hand module (1).

5. A medical instrument according to at least one of claims 1 to 4, **characterised in that** at least one, preferably two, spaced-apart plain bearing rings (24.1, 14.2) is/are placed on the cutting module (5) and cooperate(s) with one or two plain bearings (15.1, 15.2) in the hand module (1).

6. A medical instrument according to claim 5, **characterised in that** in the hand module (1) there is provided at least one plain bearing (15.1, 15.2), arranged on sliding rings (14.1, 14.2) on the cutting module (5) with a defined friction moment of at least 0.2Nm, however maximum 5 Nm.

7. A medical instrument according to claim 6, **characterised in that** the plain bearing rings (14.1, 14.2) are forced onto the cutting module (5) in a friction-locked manner.

## Revendications

1. Instrument médical pour la découpe de tissu biologique, en particulier humain, au moyen d'un module de coupe (5) avec une arête tranchante (21) qui est placé dans un module à main (1), dans lequel le module de coupe (5) est fixé de manière amovible dans le module à main (1) et établit une liaison d'encliquetage (10, 16) avec le module à main (1), dans lequel au module de coupe (5) est associée au moins une encoche (16) qui coopère avec une came d'encliquetage (10) dans le module à main (1),
**caractérisé par le fait**
**que** la came d'encliquetage (10) est réalisée sous forme d'une bague intérieure dans un élément de réception (9) et que sur le module de coupe (5) est disposée une couronne dentée (13) qui coopère avec une couronne dentée intérieure (12) sur l'élément de réception (9).

2. Instrument médical selon la revendication 1, **caractérisé par le fait que** sur l'élément de réception (9) est disposé un pignon conique (8) qui coopère avec un autre pignon conique (7) qui est placé sur un axe de rotation (6) d'un moteur (18).

3. Instrument médical selon la revendication 1 ou 2, **caractérisé par le fait que** l'au moins une encoche (16) est formée à partir du tube de coupe et réalisée de manière auto-déformable.

4. Instrument médical selon au moins l'une des revendications 1 à 3, **caractérisé par le fait que** le module de coupe (5) est un tube de coupe creux qui est connecté au module à main (1) de manière rotative autour de son axe de rotation.

5. Instrument médical selon au moins l'une des revendications 1 à 4, **caractérisé par le fait que** sur le module de coupe (5) est/sont montée(s) au moins une, de préférence deux bagues de palier lisse espacées l'une de l'autre (14.2 24.1) qui coopère(nt) avec un ou deux paliers lisses (15.1, 15.2) dans le module à main (1).

6. Instrument médical selon la revendication 5, **caractérisé par le fait que** dans le module de poignée (1) est prévu au moins un palier lisse (15.1, 15.2) qui est disposé sur des bagues coulissantes (14.1, 14.2) sur le module de coupe (5) avec un couple de friction défini d'au moins 0,2 Nm, toutefois de maximum 5 Nm.

7. Instrument médical selon la revendication 6, **caractérisé par le fait que** les bagues de palier lisse (14.1, 14.2) sont pressées en liaison de force sur le module de coupe (5).
